(19) Europäisches Patentamt
European Patent Office
Office européen des brevets

(11) **EP 1 980 319 A1**

(12) **EUROPEAN PATENT APPLICATION**
published in accordance with Art. 158(3) EPC

(43) Date of publication:
**15.10.2008 Bulletin 2008/42**

(51) Int Cl.:
**B01J 29/44** (2006.01)     **B01J 35/10** (2006.01)
**B01J 37/10** (2006.01)     **C07C 5/393** (2006.01)
**C07C 15/04** (2006.01)     **C10G 35/095** (2006.01)
**C07B 61/00** (2006.01)

(21) Application number: **07707170.2**

(22) Date of filing: **22.01.2007**

(86) International application number:
**PCT/JP2007/050907**

(87) International publication number:
**WO 2007/088745 (09.08.2007 Gazette 2007/32)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.01.2006 JP 2006022013**

(71) Applicant: **Asahi Kasei Chemicals Corporation Tokyo 101-8101 (JP)**

(72) Inventors:
• **TAKAMATSU, Yoshikazu Tokyo 100-8440 (JP)**
• **SEKIGUCHI, Mitsuhiro Tokyo 100-8440 (JP)**

(74) Representative: **von Kreisler Selting Werner Patentanwälte P.O. Box 10 22 41 50462 Köln (DE)**

(54) **CATALYST FOR PRODUCTION OF AROMATIC HYDROCARBON COMPOUND**

(57)     There is provided a zeolite-containing molded catalyst for use in production of aromatic hydrocarbon compounds by catalytic cyclization from light hydrocarbon feedstock, whereby deterioration due to precipitation of carbonaceous material during the reaction and permanent degradation due to contact with high-temperature steam during the catalyst regeneration process are suppressed to thereby allow stable production with high yield over a long period of time. In this catalyst, the zeolite contained in the zeolite-containing molded catalyst fulfills the following conditions (1), (2) and (3):

(1) the zeolite is a medium pore diameter zeolite with a pore diameter of from 5 to 6.5 A;
(2) the zeolite has a primary particle diameter in a range of from 0.02 to 0.25 μm; and
(3) the zeolite contains at least one metal element selected from the group consisting of the metal elements belonging to Group IB in the periodic table, and the zeolite-containing molded catalyst also contains at least one element selected from the group consisting of the elements belonging to Groups IB, IIB, IIIB and VIII in the periodic table.

**FIG.2**

GRAPH OF CHANGES OVER TIME IN HEXANE/HEXENE CATALYTIC CYCLIZATION REACTION USING CATALYSTS A-1, B-1 AND C-1

**Description**

Technical Field

The present invention relates to a process for producing aromatic hydrocarbon compounds from a light hydrocarbon feedstock by means of a catalytic cyclization reaction, and to a catalyst for use therein. More specifically, the present invention relates to a process for producing aromatic hydrocarbon compounds by bringing a light hydrocarbon feedstock into contact with a zeolite-containing molded catalyst, and to a zeolite-containing molded catalyst for use in this method.

Background Art

Processes for producing aromatic hydrocarbon compounds using zeolite catalysts are already known. The biggest problems with the processes of producing aromatic hydrocarbons by catalytic cyclization reactions using zeolite catalysts are first, coking deterioration, in which catalytic activity declines as carbonaceous material is deposited on the catalyst during the reaction, and second, regeneration (permanent) degradation, which occurs because a catalyst that has deteriorated due to precipitation of the carbonaceous material during industrial use needs to be regenerated, but when the carbonaceous material on the deteriorated catalyst is removed by combustion to regenerate the catalyst, aluminum in the zeolite lattice is lost due to the presence of steam occurring in the high temperature atmosphere. In recent years there have been many proposals for solving these two kinds of deterioration.

For example, Patent Document 1 discloses a method wherein IB group cations of univalent single atoms and preferably silver cations are included in the zeolite in order to improve the hydrothermal stability of the zeolite catalyst. However, Patent Document 1 does not include any examples of catalytic cyclization reactions, nor does it describe coking deterioration or zeolite particle diameter.

Patent Document 2 reports that deposition of the carbonaceous material in the reaction can be controlled and permanent degradation due to de-alumination during catalyst regeneration simultaneously prevented by using a high-silica zeolite catalyst exhibiting a specific particle diameter, ratio of surface acid site to total acid site and amount of pyridine adsorption before and after steam treatment or in other words exhibiting specific changes in acid site. However, the preferred ZSM5 zeolite in this method is synthesized by a method using seed slurry, which has poor productivity, and since the stable production range of the ZSM5 zeolite is narrow, the silica-alumina mole ratio is limited and the primary particles tend to be relatively large. According to Patent Document 2 both coking deterioration and regeneration degradation were controlled, but considering the large size of the zeolite particles, the effect on coking deterioration would not appear to be sufficient. A zeolite with a smaller primary particle diameter would be preferable, but according to Patent Document 2, in this case more carbonaceous material accumulates during the reaction, and regeneration (permanent) degradation occurs more rapidly. These circumstances are extremely problematic for industrial production of aromatic hydrocarbon compounds.

An example of a method using a proton-free zeolite is that disclosed in Patent Document 3. The catalyst used in this method effectively resists regeneration degradation, but the problem of coking deterioration remains. Consequently, coking deterioration is likely when using hydrocarbon feedstock containing many olefins. Moreover, Patent Document 3 makes no mention of the effect of particle diameter of the zeolite used in a catalytic cyclization reaction.

[Patent Document 1] Japanese Patent Application Laid-open No. S59-117584
[Patent Document 2] Japanese Patent Application Laid-open No. H10-052646
[Patent Document 3] WO 1996/13331, pamphlet

Disclosure of Invention

Problems to be Solved by the Invention

As discussed above, prior art offers no proposals for solving the twin problems of coking deterioration and regeneration degradation by simple methods. It is an object of the present invention to solve these problems.

Means for Solving the Problems

As a result of exhaustive research aimed at solving these problems, the inventors perfected the present invention upon discovering that both coking deterioration and regeneration degradation could be dramatically controlled by including one or more metals selected from the group consisting of the metals belonging to Groups IB, IIB, IIIB and VIII in the periodical table in a zeolite-containing molded catalyst used in a process of producing aromatic hydrocarbon compounds by bringing a light hydrocarbon feedstock into contact with a zeolite-containing molded catalyst, wherein the zeolite contained in the zeolite-containing molded catalyst is a medium pore diameter zeolite having a specific primary particle diameter and containing at least one metal selected from the group consisting of the metals belonging to Group IB of the periodic table. As described below, the present invention provides the process for producing the aromatic hydrocarbon compounds by the catalytic cyclization from the light hydrocarbon feedstock, along with the catalyst for use in this method.

The first aspect of the present invention provides:

[1] a zeolite-containing molded catalyst for use in a process of producing an aromatic hydrocarbon compound by catalytic cyclization from a light hydrocarbon feedstock, wherein the zeolite contained in the zeolite-containing molded catalyst fulfills the following conditions (1), (2) and (3):

(1) the zeolite is a medium pore diameter zeolite with a pore diameter of from 5 to 6.5 A;
(2) the zeolite has a primary particle diameter in a range of from 0.02 to 0.25 $\mu$m; and
(3) the zeolite contains at least one metal element selected from the group consisting of the metal elements belonging to Group IB in the periodic table, and wherein the zeolite-containing molded catalyst also comprises at least one element selected from the group consisting of the elements belonging to Groups IB, IIB, IIIB and VIII in the periodic table,

[2] the catalyst according to item [1], wherein the zeolite contains silver,
[3] the catalyst according to item [1] or [2], wherein the zeolite is an MFI zeolite,
[4] the catalyst according to any one of items [1] to [3], wherein the zeolite-containing molded catalyst is heat treated at 500°C or more in the presence of steam prior to contact with the light hydrocarbon feedstock.

Moreover, the second aspect of the invention provides:

[5] a process for producing an aromatic hydrocarbon compound, comprising bringing a light hydrocarbon feedstock into contact with a zeolite-containing molded catalyst according to any one of items [1] to [4],
[6] the process according to item [5], wherein a reactor used in the process is an adiabatic fixed-bed reactor.

Advantageous Effects of the Invention

With the zeolite-containing molded catalyst and aromatic hydrocarbon compound producing process according to the present invention, it is possible to stably produce aromatic hydrocarbon compounds with high yield by catalytic cyclization from the light hydrocarbon feedstock. The zeolite-containing molded catalyst used in the producing process according to the present invention is highly resistant to coking deterioration and regeneration degradation. These properties are extremely useful for industrial application in the production of aromatic hydrocarbon compounds.

Best Mode for Carrying Out the Invention

The present invention is explained in detail below.
A so-called "medium pore diameter zeolite" with a pore diameter of from 5 to 6.5 A is used as the zeolite contained in the zeolite-containing molded catalyst according to the present invention. As used in the present invention, the term "medium pore diameter zeolite" means "a zeolite with a range of pore diameters between the pore diameters of small-pore zeolites (typically A-type zeolites) and the pore diameters of large-pore zeolites (typically mordenite and X-type and Y-type zeolites)", and this zeolite has an 10-membered oxygen ring in the crystal structure.

Examples of the medium pore diameter zeolites may include ZSM-5 and so-called pentasil zeolites, which are structurally similar to ZSM-5. These include ZSM-5, ZSM-8, ZSM-11, ZSM-12, ZSM-18, ZSM-23, ZSM-35, ZSM-39 or the like. Of these zeolites, the most desirable types of zeolites are those represented as MFI structures according to the IUPAC nomenclature for zeolite frameworks, and ZSM-5 is particular desirable.

A zeolite containing at least one metal selected from the group consisting of metals belonging to Group IB of the periodic table (hereinafter referred to as "Group IB metals"), or in other words from the group consisting of copper, silver and gold, is used as the zeolite. Of the Group IB metals, copper and silver are preferred, and silver is especially preferred. In this description, the term "periodic table" means the periodic table described on pages 1-15 of the CRC Handbook of Chemistry and Physics (75th edition), David R. Lide et al., CRC Press Inc. (1994-1995).

The term "zeolite contains at least one metal element selected from the group consisting of the metal elements belonging to Group IB in the periodic table" means that the zeolite contains the Group IB metal in the form of the corresponding cations.

In the zeolite-containing molded catalyst according to the present invention, Group IB metal cations carried by ion exchange on the zeolite are a cause of cracking activity.

Examples of methods for incorporating the Group IB metal element into the zeolite may include methods in which a zeolite containing no Group IB metal is treated by the known ion-exchange method to incorporate the metal element, such as a liquid-phase ion-exchange treatment method or a solid-phase ion exchange treatment method in which the impregnated catalyst is subjected to high-temperature treatment or the like. When the Group IB metal is incorporated into the zeolite by such the ion-exchange method, a salt of the Group IB metal must be used. Examples of Group IB metal salts may include silver nitrate, silver acetate, silver sulfate, copper chloride, copper sulfate, copper nitrate and gold chloride. Silver nitrate or copper nitrate is preferred, and silver nitrate is especially preferred.

The content of the Group IB metal in the zeolite-containing molded catalyst as IB metal cations is not strictly limited, but since the silica-alumina mole ratio ($SiO_2/Al_2O_3$ mole ratio) of the zeolite used is from 60 to 200, and since the metal is carried by ion exchange, the content of the Group IB metal is naturally determined by the exchange capacity and the zeolite content of the zeolite-containing molded catalyst. Therefore, the exchange rate of the Group IB metal cations as a percentage of exchange sites in the zeolite should be in the range of generally from 5% to 95%, preferably from 30% to 90%, more preferably from 50 to 85%, since if the exchange rate is too low activity will be insufficient, while if the exchange rate is too high the ion-exchange preparation step will be too difficult. Note that the content of the Group IB metal in the zeolite can be determined by the known method such as x-ray fluorescence analysis.

As well as being included in the zeolite as cations, the Group 1 B metal in the zeolite-containing molded catalyst may also be included in a form other than cations as discussed below, such as an oxide form. As discussed below this additional Group IB metal contributes strong dehydrogenation ability to the catalyst according to the present invention, and is included in order to improve the yield of aromatic hydrocarbon compounds.

The ion-exchange sites that are not exchanged with Group IB metal cations in the zeolite contained in the zeolite-containing molded catalyst according to the present invention are of course exchanged with protons or metal cations, and are preferably exchanged with alkali metal cations or metal cations belonging to Group IIB, IIIB or VIII.

The silica-alumina mole ratio of the zeolite is preferably at least 60 but no more than 200. If the silica-alumina mole ratio is less than 60 the catalyst will be somewhat less stable with respect to high-temperature steam and less resistant to regeneration degradation, which is undesirable because the activity is likely to decline gradually in the course of repeated reaction and regeneration in the case of industrial application to the production of aromatic hydrocarbon compounds.

If the silica-alumina mole ratio is over 200, however, the carried amount of the Group IB metal will be insufficient, detracting from cracking activity and from aromatic hydrocarbon yield. Moreover, ion-exchange rate of the zeolite must be increased in order to adjust the Group IB metal content proportionately so as to maintain the catalytic activity of the zeolite-containing molded catalyst of high silica-alumina ratio, but this makes the ion-exchange rate efficiency of the Group IB metal lower and catalyst preparation more difficult, and is highly impractical for industrial purposes. More preferably, the silica-alumina mole ratio of the zeolite is at least 80 but no more than 120. The silica-alumina mole ratio of the zeolite can be determined by the known method, such as for example a method in which the zeolite is completely dissolved in an aqueous alkali solution or aqueous hydrofluoric acid solution, and the resulting solution is analyzed by plasma emission spectrometry or the like to determine the ratio.

A metalloaluminosilicate in which some of the aluminum atoms in the zeolite framework are replaced by atoms of Ga, Fe, B, Cr or the like, or a metallosilicate in which all the aluminum atoms in the zeolite framework are replaced by such atoms, can also be used as the zeolite. In this case, the silica-alumina mole ratio is calculated after converting the contents of the aforementioned elements in the metalloaluminosilicate or metallosilicate into moles of alumina.

The primary particle diameter of the zeolite is in the range of from 0.02 to 0.25 $\mu$m. Preferably, the primary particle diameter is in the range of from 0.02 to 0.2 $\mu$m, more preferably from 0.03 to 0.15 $\mu$m. The primary particles of zeolite may be present as individual particles or as secondary aggregates. Since in most cases the primary particles aggregate to form secondary particles, and since the primary particles assume various forms, a method of measuring the Feret diameter (see The Society of Chemical Engineers, Japan Ed., Kagaku Kogaku Binran (Chemical Engineering Handbook), Revised 6th Edition, p. 233) from a scanning microscope image of zeolite powder taken at 100,000x magnification can be adopted for measuring the primary particle diameter in the present invention. Primary particles having this particle diameter should preferably constitute at least 50 % by mass, more preferably at least 80 % by mass of the total.

The smaller the particle diameter of the zeolite, the greater the effective surface area, which is known to be an advantage in terms of both activity and coking deterioration. The particle diameter of the zeolite is influenced not by the particle diameter of secondary particles formed by aggregation of primary particles, but by the particle diameter of the primary particles that can be distinguished at 100,000x magnification under a scanning electron microscope. Consequently, the Feret diameter of the primary particles of zeolite as measured by scanning electron microscopy of zeolite powder at 100,000x magnification is preferably in the range of from 0.02 to 0.25 $\mu$m.

However, the crystal structure of such a fine-particle zeolite is unstable, the lattice aluminum is easily detached by high-temperature treatment in the presence of steam as described in Patent Document 2, and such poor hydrothermal stability is likely to cause regeneration (permanent) degradation. Surprisingly, however, with the zeolite-containing molded catalyst used in the present invention hydrothermal stability is much improved even in the case of a fine-particle zeolite, and regeneration degradation can be greatly controlled, as can the amount of carbonaceous material accumulation. According to the present invention, the zeolite-containing molded catalyst is achieved which is resistant to both the conventional problems of coking deterioration and regeneration deterioration.

At least one element selected from the group consisting of the elements belonging to Groups IB, IIB, IIIB and VIII in the periodic table is included in the zeolite and / or the zeolite-containing molded catalyst in order to confer strong dehydrogenation ability on the zeolite-containing molded catalyst according to the present invention. The metals of copper, zinc, gallium, indium, nickel, palladium and platinum and oxides and complex oxides thereof are preferred, and zinc and zinc compounds are more preferred.

Ion exchange or impregnation is generally used as the method of incorporating metal elements and compounds of metal elements belonging to Groups IB, IIB, IIIB and VIII in the periodic table into the zeolite and / or zeolite-containing molded catalyst in order to confer strong dehydrogenation ability on the zeolite containing molded catalyst according to the present invention.

The amount of the metal elements and compounds of metal elements belonging to Groups IB, IIB, IIIB and VIII in the periodic table that is incorporated into the zeolite and / or zeolite-containing molded catalyst in order to confer strong dehydrogenation ability on the zeolite-containing molded catalyst according to the present invention is generally from 0.1 to 25 % by mass, preferably from 5 to 20 % by mass as elements.

In the zeolite-containing molded catalyst according to the present invention, the binder or molding diluent (matrix) is generally a porous, flame-resistant inorganic oxide such as alumina, silica, silica / alumina, zirconia, titania, diatomaceous earth or clay. Alumina or silica is preferred, and alumina is especially preferred. This is mixed with the zeolite described above, the mixture is molded, and the resulting molded body is used as the zeolite-containing molded catalyst. When using the matrix or binder, the content thereof is preferably in the range of from 5 to 90 % by mass, more preferably from 5 to 50 % by mass, based on the total weight of the zeolite and the matrix or binder.

The zeolite-containing molded catalyst according to the present invention can be subjected to heat treatment at 500°C or more in the presence of steam prior to contact with the light hydrocarbon feedstock with the aim of improving resistance to coking deterioration. Heat treatment is preferably performed at a temperature of at least 500°C but no more than 900°C, under a steam partial pressure of 0.01 atm or more.

In a preferred example of the present invention the zeolite-containing molded catalyst according to the present invention contains a mixture of a Group IB metal-exchanged zeolite, zinc and a compound thereof and alumina, and in this case the high-temperature steam treatment serves to stabilize the zinc component in the catalyst as zinc aluminate, thereby achieving the additional object of greatly controlling scattering loss of zinc in the reaction atmosphere. This effect is extremely advantageous for industrial application to the production of the aromatic hydrocarbon compounds. The zinc aluminate discussed in the description of the present application has an x-ray diffraction pattern identical to the pattern given in JCPDS 5-0669NBS Circ., 539, Vol. II, 38(1953).

Aromatic hydrocarbon compounds can be obtained by packing such a specific zeolite-containing molded catalyst into a reactor, and bringing it into contact with the light hydrocarbon feedstock to thereby perform the catalytic cyclization reaction. The aromatic hydrocarbon compounds can be separated and collected by the known methods from the resulting reaction mixture.

The light hydrocarbon feedstock is a light hydrocarbon feedstock containing at least one selected from the olefins and paraffins, wherein the hydrocarbons have 2 or more carbon atoms and a 90 % distillation temperature of 190° or less. Examples of such paraffins may include ethane, propane, butane, pentane, hexane, heptane, octane and nonane.

Examples of such olefins may include ethylene, propylene, butene, pentene, hexene, heptene, octene and nonene. In addition, cyclopentane, methylcyclopentane, cyclohexane and other cycloparaffins, cyclopentene, methylcyclopentene, cyclohexene and other cycloolefins and / or cyclohexadiene, butadiene, pentadiene, cyclopentadiene and other dienes may be included.

A mixture of light hydrocarbons may be used as the raw material, or methane, hydrogen or an inert gas such as nitrogen, carbon dioxide, carbon monoxide or the like may be included in the mixture as a diluent.

These diluents may constitute preferably 20% or less, more preferably 10% or less by volume. It is particularly desirable to use a mixture containing saturated hydrocarbons and unsaturated hydrocarbons at a weight ratio of between 1 / 0.33 and 1 / 2.33. The weight ratio of saturated hydrocarbons to unsaturated hydrocarbons here means the weight ratio in the supplied mixture.

For the light hydrocarbon feedstock, it is possible to use the aforementioned hydrocarbon mixture, or the $C_4$ fraction of a high-temperature pyrolysis product of a petroleum hydrocarbon such as naphtha, a fraction obtained by removing the butadiene and isobutylene from such a $C_4$ fraction, the $C_5$ fraction of a high-temperature pyrolysis product of a petroleum hydrocarbon, a fraction obtained by removing the dienes from such a $C_5$ fraction, thermally cracked gasoline, a rafinate obtained by extracting aromatic hydrocarbons from thermally cracked gasoline, a rafinate obtained by extracting aromatic hydrocarbons from FCC-LPG, FCC cracked gasoline or reformate, or coker LPG, straight-run naphtha or the like, but of these, it is particularly desirable to use the $C_4$ or $C_5$ fraction of a high-temperature pyrolysis product of a petroleum hydrocarbon such as naphtha or a fraction obtained by removing some or all of the butadiene, isobutylene, isoprene and cyclopentadiene from such a fraction, and a raw material in which the weight ratio of the $C_4$ fraction to the $C_5$ fraction is 3/7 to 7/3 is particularly desirable. The weight ratio of the $C_4$ fraction to the $C_5$ fraction here means the weight ratio of the supplied mixture.

The light hydrocarbon feedstock may also contain, as impurities, *tert*-butyl alcohol, methyl *tert*-butyl ether, methanol and other oxygen-containing compounds.

The conditions for the catalytic cyclization reaction vary according to the light hydrocarbon feedstock and particularly according to the relative amounts of olefins and paraffins in the feedstock, but a temperature of from 300 to 650°C, a hydrocarbon partial pressure of between atmospheric pressure and 30 atm, and a weight hourly space velocity (WHSV) of from 0.1 to 50 hr$^{-1}$ based on the weight of the molded catalyst are preferred. More preferably, the reaction temperature is in the range of from 400 to 600°C.

A fixed-bed reactor, moving-bed reactor, fluidized-bed reactor or stream transport system can be used in the present invention as the reactor for catalytic cyclization of the light hydrocarbon feedstock using the zeolite-containing molded catalyst, a structurally simple adiabatic fixed-bed reactor is preferred.

The zeolite-containing molded catalyst may suffer coking deterioration if used in catalytic conversion for a long period of time, but in this case the deteriorated catalyst can be regenerated by burning off the coke on the catalyst at a temperature of from 400 to 700°C, usually in an atmosphere of air or a gaseous mixture of oxygen and an inert gas (this treatment is hereinafter referred to as "catalyst regeneration process").

Because the zeolite-containing molded catalyst according to the present invention is resistant to deterioration due to coking, aromatic hydrocarbon compounds can be stably produced over a long period of time even using a fixed-bed reactor. Since the zeolite-containing molded catalyst according to the present invention is also highly resistant to de-alumination in the presence of high-temperature steam, it undergoes very little permanent degradation (regeneration degradation) during the catalyst regeneration process, and consequently suffers very little loss of activity even after repeated reaction and regeneration. Therefore, aromatic hydrocarbon compounds can be produced stably and with high yield over a long period of time. These features are extremely useful for industrial application in the production of aromatic hydrocarbon compounds.

Examples

The present invention is explained in more detail below by means of examples and comparative examples, but the present invention is in no way limited by these examples.
Note that measurements in the examples and comparative examples were performed as follows.

(1) Measurement of silica-alumina mole ratio of zeolite

0.2 g of zeolite powder sample was taken in a Teflon® vessel, and after addition of 6 ml of nitric acid (68% ultrapure grade) and 1 ml of fluoric acid (ultrapure grade), was decomposed and dissolved with a Milestone General Ethos Plus microwave labstation. Once dissolution was complete, pure water was added to a total of 20 g. The resulting zeolite solution was diluted with ion-exchanged water, the silicon and aluminum concentrations in the diluted liquid were measured with a plasma spectrometer (ICP device), and the silica-alumina mole ratio of the zeolite was calculated from the results.

ICP device and measurement conditions

Device: Jobin Yvon (JY138 Ultrace), Rigaku Corp.

Measurement conditions:

| Silicon measurement wavelength | 251.60 nm |
| Aluminum measurement wavelength | 396.152 nm |
| Plasma power | 1.0 kw |
| Nebulizer gas | 0.28 L / min |
| Sheath gas | 0.3 to 0.8 L / min |
| Coolant gas | 13L / min |

(2) Measurement of zeolite primary particle diameter

A zeolite powder sample was held with carbon adhesive tape on an aluminum sample platform, and subjected to Pt deposition with an ion sputterer (Hitachi E-1030) to maintain conductivity.

SEM images were taken with a Hitachi FE-SEM (S-800), at an acceleration voltage (HV) of 20 KV at magnifications of 500, 15,000 and 100,000.

Secondary particles are often formed by aggregation of fine primary particles in the zeolite used in the present invention, so the Feret diameters of 20 or more primary particles that were determined to be primary particles because they appeared as single masses without cracks in the 100,000x scanning electron microscope image were measured, and the average was given as the primary particle diameter. When the primary particle diameter was so large that it could not be evaluated in a 100,000x scanning electron microscope image, the scanning electron microscope image of the different magnification was selected appropriately for purposes of comparison, and the primary particle diameter was determined in the same way.

[Example 1]

(Catalyst preparation)

The primary particle diameter of an H-type ZSM-5 zeolite power with a silica-alumina mole ratio of 92 was 0.06 μm as measured from the scanning electron microscope image taken at a magnification of 100,000x.

1.3 kg of zinc nitrate hexahydrate and alumina sol were blended with 2 kg of this zeolite powder so as to achieve a zeolite / alumina ratio of 8 / 2. This was mixed and kneaded with the moisture content adjusted as necessary, and extrusion molded to 1 / 16" x 5 to 10 mm. The resulting extrusion molded catalyst was dried for 12 hours at 120°C, and then burned for 6 hours at 500°C. Next, the resulting zinc-carrying zeolite-containing molded catalyst was dispersed in 1 N sodium nitrate aqueous solution (10 cc / g-molded zeolite), and subjected three times to ion-exchange treatment for 1 hour at room temperature. This was then filtered, water washed and dried. This was then dispersed in 0.1 N silver nitrate aqueous solution (10 cc / g-molded zeolite), and subjected to ion-exchange treatment for 2 hours at room temperature. This was then filtered, water washed and dried to prepare catalyst A.

The Ag content of catalyst A as measured by fluorescent x-ray analysis was 1.80 % by mass based on 100 % by mass of catalyst A. The ion exchange rate of silver cations relative to zeolite exchange sites was estimated to be 66%.

(Steam treatment)

100 g of catalyst A was packed in a Hastelloy reactor tube with an inner diameter of 27.2 mm, heated to 650°C in a flow of nitrogen at atmospheric pressure, and then steamed for 3 hours under conditions of steam flow rate 218 g / Hr, nitrogen flow rate 220 NL / Hr to obtain catalyst A-1. A similar steam treatment was also performed but with a processing time of 10 hours to obtain catalyst A-2.

(Catalytic cyclization reaction test)

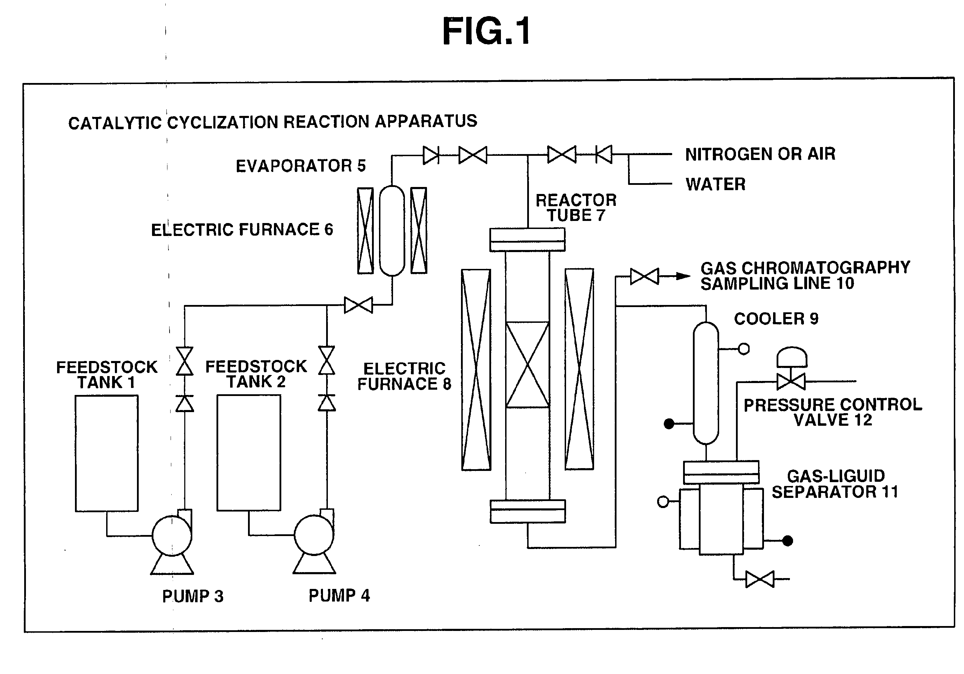

A catalytic cyclization reaction was performed using the reaction apparatus shown in FIG. 1 and a mixed feedstock having an n-hexane / 1-hexene weight ratio of 1 / 1. 60 g of catalyst A-1 was packed in Hastelloy reactor 7 having an inner diameter of 27.2 mm. The feedstock was mixed in feedstock tank 1, and supplied to the reactor by pump 3 at a supply rate of 300 g / Hr while being vaporized via evaporator 5. The temperature of the reactor catalyst layer was controlled at 515°C by means of electric furnace 8. The reactor outlet gas was cooled by cooler 9 and separated into gas and liquid by gas-liquid separator 11, and the gas component was exhausted outside the system via pressure control valve 12. A pressure of 0.5 MPa / G was maintained inside the system by means of pressure control valve 12. The system was also heated with a line heater so as to maintain a gaseous phase up to the gas chromatography sampling line 10 at the reactor outlet so that all of the reactor outlet components could be sampled and analyzed as a gaseous phase.

A predetermined time after the supply of feedstock was initiated, the reaction product was introduced directly from the reactor outlet to a gas chromatography (TCD, FID detector) and the composition was analyzed.

The conditions for gas chromatography analysis were as follows.

Device: Shimadzu GC-17A

Column: Supelco (U.S.) SPB-1 custom capillary column (inner diameter 0.25 mm, length 60 m, film thickness 3.0 μm)

Sample gas volume: 1 mL (sampling line maintained at 200 to 300°C)

Temperature program: Maintained for 12 minutes at 40°C, then raised to 200°C at 5°C / minute, then maintained for 22 minutes at 200°C

Split ratio: 200 : 1

Carrier gas (nitrogen) flow rate: 120 mL / minute

FID detector: Air supply pressure 50 kPa (about 500 mL / minute), hydrogen supply pressure 60 kPa (about 50 mL / minute)

Measurement method: A TCD detector and FID detector were connected in a line, hydrogen and $C_1$ and $C_2$ hydrocarbons were detected with the TCD detector, and $C_{3+}$ hydrocarbons were detected with the FID detector. 10 Minutes after the start of analysis, detector output was switched from TCD to FID.

The reaction product was analyzed as appropriate while the reaction was performed continuously for 48 hours. A similar reaction was performed using catalyst A-2.

The resistance of the catalyst to regeneration degradation was evaluated in terms of a numerical value obtained from the following Formula (1), which shows the decrease in activity in relation to steaming time. That is, activity at the start of the reaction was substituted in the method disclosed in Patent Document 2 above for evaluating by means of changes in pyridine adsorption amount.

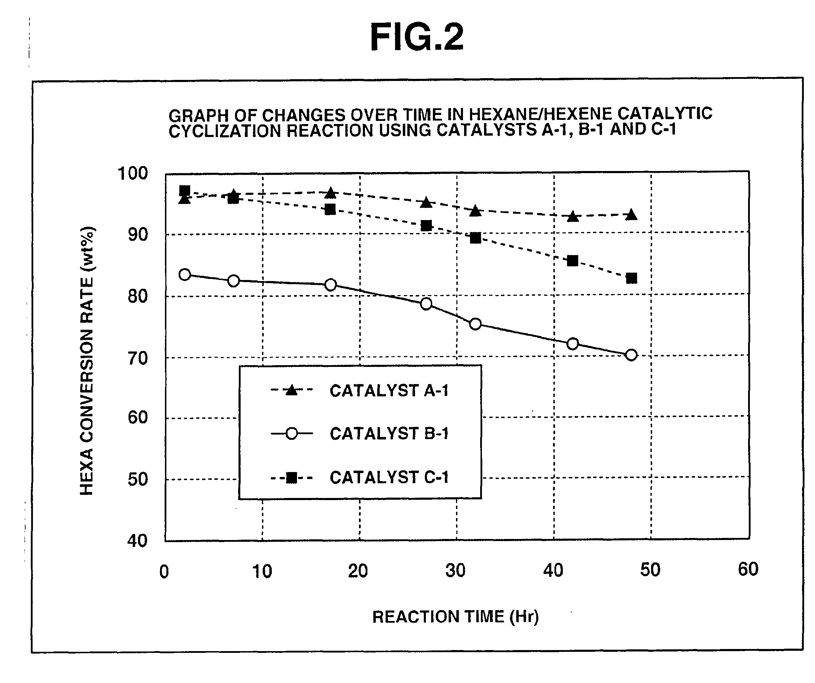

The results are shown in Table 1 and FIG. 2.

$$\text{Regeneration degradation index } C = (1\,/\,B^2 - 1\,/\,A^2) \times 1000 \qquad (1)$$

wherein

B = initial reaction rate constant K(2) at 10 hrs of steam treatment

A = initial reaction rate constant K(2) at 3 hrs of steam treatment

K(2): reaction rate constant at 2 hours (initial activity)

K (primary reaction rate constant based on hexane) = WHSVxLn (1 / (1 - conv / 100)).

The resistance of the catalyst to coking deterioration was evaluated in terms of the coking deterioration rate constant D, which was determined by the following Formula (2).

$$K(48)\,/\,K(2) = EXP(-D^*t) \qquad (2)$$

wherein

D:        coking deterioration rate constant

K(48):    reaction rate constant at 48 hours

K(2):     reaction rate constant at 2 hours (initial activity)

t:        reaction time (Hr).

Fine-particle zeolites are conventionally known for having unstable crystal structures and poor hydrothermal stability, but it can be seen from this example and from Comparative Examples 1 and 2 below that despite containing a fine-particle zeolite, the zeolite-containing molded catalyst according to the present invention has extremely good hydrothermal stability and is highly resistant to regeneration degradation. The zeolite-containing molded catalyst according to the present invention also exhibits strong catalytic cyclization activity despite having a somewhat high silica-alumina ratio. Of course, this is because there is little loss of activity even after the steaming pre-treatment that is necessary to control coking deterioration.

[Comparative Example 1]

An H-type ZSM-5 zeolite was synthesized by the methods described in Example 1 of the specification of Patent Document 2. The resulting zeolite has a silica-alumina mole ratio of 42. A powder of this zeolite had a primary particle diameter of 1.54 $\mu$m as measured by scanning electron microscopy at a magnification of 15,000x.

An H-ZSM-5 zeolite-containing molded catalyst containing 10 % by mass of zinc, catalyst B, was obtained by the same methods as in Example 1.

Catalyst B was subjected to steam treatment by the same methods as in Example 1 to prepare a 3-hour treated catalyst (B-1) and a 10-hour treated catalyst (B-2).

Reactions were performed in the same way as the catalytic cyclization reaction test of Example 1, but using catalysts B-1 and B-2. The results are shown in Table 1 and FIG. 2.

It can be seen from Comparative Example 1 that with the conventional H-type zeolite system, even if the zeolite (hereinafter simply referred to as "zeolite of Patent Document 2") has the physical properties stipulated in the invention of Patent Document 2, hydrothermal stability is much less than with the catalyst according to the present invention. That is, it appears that using the zeolite of Patent Document 2, permanent degradation (regeneration degradation) is likely to progress over time in the course of industrial use. Moreover, the particle diameter of the zeolite of Patent Document 2 is larger than the primary particle diameter of the zeolite used in the zeolite-containing molded catalyst according to the present invention, and consequently the rate of deterioration due to precipitation of carbonaceous material during the reaction (coking deterioration) is much more rapid than with the zeolite-containing molded catalyst according to the present invention.

[Comparative Example 2]

Na ion exchange and Ag ion exchange of catalyst B were performed by the same ion exchange methods as in Example 1 to prepare catalyst C. The Ag content of catalyst C was 1.87 % by mass based on 100 % by mass of catalyst C. Catalyst C was steam treated as in Example 1 to prepare a 3-hour treated catalyst (C-1) and a 10-hour treated catalyst (C-2).
Reactions were performed in the same way as the catalytic cyclization reaction test of Example 1, but using catalysts C-1 and C-2. The results are shown in Table 1 and FIG. 2.
It can be seen from Comparative Example 2 that in comparison with the H-type catalyst, hydrothermal stability is greatly improved by using the zeolite of Patent Document 2 with silver carried thereon by ion exchange. However, coking deterioration is still much greater than with the catalyst according to the present invention.

[Example 2]

(Catalyst preparation)

1.25 kg of zinc nitrate hexahydrate and alumina sol were blended with 3 kg of the H-type ZSM-5 zeolite powder with a silica-alumina mole ratio of 92 that was used in Example 1 so as to achieve a zeolite / alumina ratio of 9 / 1 (7.5 % by mass as zinc relative to zeolite-containing molded catalyst). This was mixed and kneaded with the moisture content adjusted as necessary, and extrusion molded to 1 / 16" x 5 to 10 mm. The resulting extrusion molded catalyst was dried for 12 hours at 120°C, and then burned for 6 hours at 500°C to obtain a zinc-carrying zeolite-containing molded catalyst. Next, 1 kg of the resulting zinc-carrying zeolite-containing molded catalyst was immersed in an aqueous silver nitrate solution containing 30 g of silver nitrate dissolved in 500 g of ion-exchanged water, and the moisture was then distilled off with a rotary evaporator at a bath temperature of 60°C and a pressure of 55 torr. The dried molded catalyst was collected, dried for 5 hours at 120°C, and burned for 15 hours at 500°C to obtain catalyst D.
1.25 kg of zinc nitrate hexahydrate, 120 g of silver nitrate and alumina sol were blended with 3 kg of the H-type ZSM-5 zeolite powder with a silica-alumina mole ratio of 92 that was used in Example 1 so as to achieve a zeolite / alumina ratio of 9 / 1. This was mixed and kneaded with the moisture content adjusted as necessary, and extrusion molded to 1 / 16" x 5 to 10 mm. The resulting extrusion molded catalyst was dried for 12 hours at 120°C, and then burned for 15 hours at 500°C to obtain catalyst E.
The Ag contents of catalysts D and E as measured by fluorescence x-ray analysis were 1.85 and 1.96 % by mass, respectively, based on 100 % by mass of catalysts D and E, while the zinc contents were 7.32 and 7.22 % y mass, respectively. Catalysts D and E were also crushed and powdered, added to 1 mol / L aqueous sodium nitrate solutions, and stirred for 3 hours at 60°C. The solids were filtered out, the Ag contents in the solutions were measured with the ICP device, and based on these measurements, the Ag concentrations of catalysts D and E were found to be 1.75 and 1.88 % by mass, respectively. This shows that with the preparation methods of these examples, the silver is contained in the zeolite exchange sites in the form of cations.

(Steam treatment)

Catalysts D and E were steam treated for 3 hours at 650°C by the same method as in Example 1 to obtain Catalysts D-1 and E-1.

(Catalytic cyclization reaction tests)

Catalytic cyclization reaction tests were performed as in Example 1 except that the catalyst packing volume was 10 g, the feedstock supply rate was 150 g / Hr and the reaction time was 36 hours. The results of reaction tests performed using Catalyst A-1, Catalyst D-1 and Catalyst E-1 are shown in Table 2.

[Example 3]

A catalytic cyclization reaction test was performed with catalyst A-1 (60 g) using the same apparatus as in Example 1.

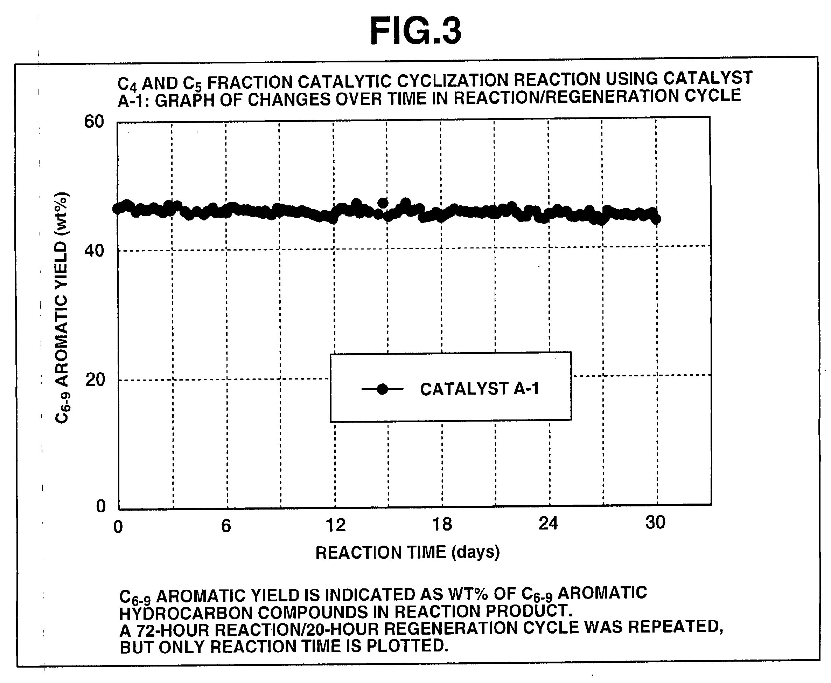

For the raw material light hydrocarbon feedstock, the $C_4$ fraction and $C_5$ fraction shown in Table 3 were supplied by pumps 3 and 4 from raw material tanks 1 and 2, respectively, mixed and used. The weight ratio of the $C_4$ fraction to the $C_5$ fraction was 4:6. The weight ratio of unsaturated hydrocarbons to saturated hydrocarbons in this mixed light hydrocarbon feedstock was 47.8 / 52.2. The raw material supply rate was 168 g / Hr (WHSV = 2.8).
The reaction temperature was 515°C, the reaction pressure was 0.5 MPa / G and the reaction time was 72 hours.
After completion of the reaction, the catalyst was regenerated by the following procedures as the catalyst regeneration process. Nitrogen was substituted as the catalyst was heated to 420°C over the course of 2 hours, and nitrogen diluted gas with an oxygen concentration of 1 % was then substituted to initiate regeneration. The CO and $CO_2$ concentrations of the outlet gas were monitored as the temperature and oxygen concentration were gradually raised, and nitrogen was substituted once the outlet $CO_2$ concentration was at or below the detection limit at the final oxygen concentration of 5% and temperature of 550°C, completing the catalyst regeneration process. The time required for the catalyst regeneration process was 20 to 24 hours. This cycle of 72 hours of reaction followed by 20 to 24 hours of regeneration was repeated 10 times in continuous operation.
The results are shown in FIG. 3.
It can be seen that using the zeolite-containing molded catalyst according to the present invention, three days of continuous operation can be accomplished without difficulty and without adversely affecting deterioration, the amount of carbonaceous material that accumulates during the reaction can be removed in a one-day catalyst regeneration process, and the initial activity of the catalyst is not diminished by repeating the reaction 10 times.

[Example 4]

The primary particle diameter of an H-type ZSM-5 zeolite powder with a silica-alumina mole ratio of 83 was 0.13 $\mu$m as measured with the scanning electron microscope at a magnification of 100,000x.
1.3 kg of zinc nitrate hexahydrate and alumina sol were blended with 2 kg of this zeolite powder so as to obtain a zeolite / alumina ratio of 8 / 2. This was mixed and kneaded with the moisture content adjusted as necessary, and extrusion molded to 1 / 16" x 5 to 10 mm. The resulting extrusion molded catalyst was dried for 12 hours at 120°C, and then burned for 6 hours at 500°C.
The resulting zinc-carrying zeolite-containing molded catalyst was dispersed in a 1 N sodium nitrate aqueous solution (10 cc / g-molded zeolite), and subjected three times to a 1-hour ion-exchange treatment at room temperature. This was then filtered, water washed and dried. This was dispersed in 0.1 N silver nitrate aqueous solution (10 cc / g-molded zeolite), and ion-exchange treated for 2 hours at room temperature. This was then filtered, water washed and dried to prepare catalyst F.
The Ag content of catalyst F as measured by fluorescence x-ray analysis was 1.77 % by mass based on 100 % by mass of catalyst D.

(Steam treatment)

100 g of catalyst F was packed in a Hastelloy reaction tube with an inner diameter of 27.2 mm, heated to 650°C in a flow of nitrogen at atmospheric pressure, and then steamed for 3 hours under conditions of steam flow rate 218 g / Hr, nitrogen flow rate 220 NL / Hr to obtain catalyst F-1.

(Catalytic cyclization reaction test)

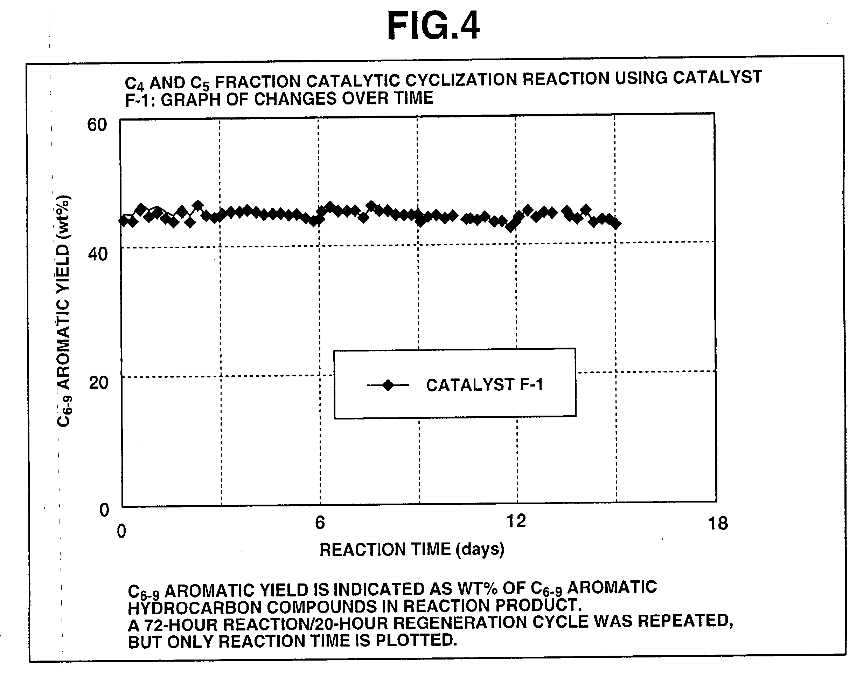

A catalytic cyclization reaction test was performed as in Example 2, but using F-1 as the catalyst. The 72-hour reaction cycle was repeated five times. The results are shown in FIG. 4.

[Comparative Example 3]

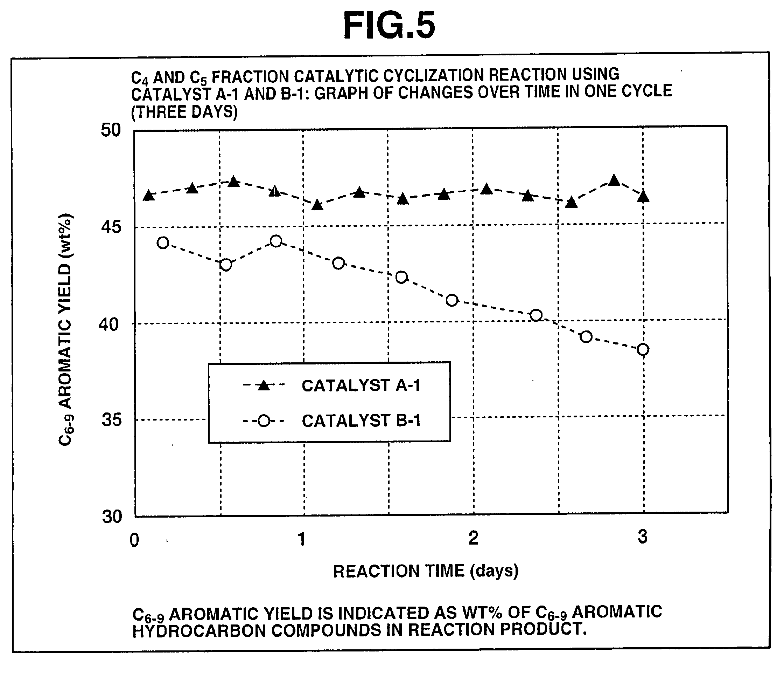

Only one cycle of a catalytic cyclization reaction test was performed as in Example 2 but using B-1 as the catalyst. The results are shown in FIG. 5 together with the results of Example 2, and it can be seen that the yield is somewhat lower while coking deterioration is greater.
Judging from Examples 2 and 3 and this comparative example, the zeolite-containing molded catalyst in the process according to the present invention provides much better hydrothermal stability in a fine-particle zeolite than conventionally proposed H-type zeolite catalysts. Consequently, it is possible to greatly control the conventional problems of regeneration degradation and coking deterioration so that aromatic hydrocarbon compounds can be produced stably and with high yield over a long period of time.

[Table 1]

[Table 2]

# TABLE 1

## CATALYTIC CYCLIZATION REACTION TEST RESULTS

| | STEAM TREATMENT TIME | C6P CONVERSION RATE (wt%) | | C69 AROMA YIELD (wt%) | | INITIAL ACTIVITY (Hr$^{-1}$) | COKING DETERIORATION RATE CONSTANT D ($*10^{-3}$) | REGENERATION DETERIORATION INDEX C |
|---|---|---|---|---|---|---|---|---|
| | | 2Hr | 48Hr | 2Hr | 48Hr | | | |
| CATALYST A-1 | 3Hr | 96.4 | 93.3 | 45.2 | 44.3 | 16.62 | 4.33 | 2.98 |
| CATALYST A-2 | 10Hr | 91.5 | 87.5 | 44.2 | 42.9 | 12.31 | 3.50 | |
| CATALYST B-1 | 3Hr | 83.4 | 70.3 | 34.6 | 32.5 | 8.98 | 8.14 | 5.97 |
| CATALYST B-2 | 10Hr | 77.1 | 60.0 | 31.2 | 28.2 | 7.38 | 9.94 | |
| CATALYST C-1 | 3Hr | 97.0 | 82.8 | 45.3 | 39.4 | 17.60 | 14.41 | 2.61 |
| CATALYST C-2 | 10Hr | 92.7 | 83.1 | 42.5 | 39.7 | 13.09 | 8.05 | |

C6P CONVERSION RATE: CONVERSION RATE OF HEXANE IN FEEDSTOCK SHOWN AS WT %

C69 AROMA YIELD: $C_{6-9}$ AROMATIC HYDROCARBON COMPOUNDS IN REACTION PRODUCT SHOWN AS WT %

PRIMARY REACTION RATE CONSTANT BASED ON HEXANE: $K = WHSV * Ln (1/(1-conv/100))$

REGENERATION DETERIORATION INDEX C: $C = (1/B^2 - 1/A^2) * 1000$    B = INITIAL REACTION RATE CONSTANT WITH 10Hrs STEAM TREATMENT

A = INITIAL REACTION RATE CONSTANT WITH 3Hrs STEAM TREATMENT

COKING DETERIORATION RATE CONSTANT D: $K (48)/K (2) = EXP (-D * t)$    K (48) : REACTION RATE CONSTANT AT 48 HOURS

K (2)  : REACTION RATE CONSTANT AT 2 HOURS (INITIAL ACTIVITY)

t : REACTION TIME (Hr)

EP 1 980 319 A1

**TABLE 2:**

| CATALYTIC CYCLIZATION REACTION TEST RESULTS | | | | |
|---|---|---|---|---|
| | C6P CONVERSION RATE (wt%) | | C69 AROMA YIELD (wt%) | | COKING DETERIORATION RATE CONSTANT D |
| | 2Hr | 48Hr | 2Hr | 48Hr | |
| CATALYST A-1 | 75.6 | 50.2 | 37.6 | 28.9 | 0.020 |
| CATALYST D-1 | 77.5 | 52.8 | 38.7 | 30.9 | 0.019 |
| CATALYST E-1 | 76.8 | 51.2 | 37.9 | 29.7 | 0.020 |

[Table 3]

**TABLE 3:**

| COMPOSITION OF LIGHT HYDROCARBON FEEDSTOCK | | |
|---|---|---|
| COMPONENT | $C_4$ FRACTION HYDROCARBON COMPOSITION (wt%) | $C_5$ FRACTION HYDROCARBON COMPOSITION (wt%) |
| $C_3H_8$ | 0.1 | 0.0 |
| $C_3H_6$ | 0.1 | 0.0 |
| $C_4H_{10}$ | 17.2 | 0.0 |
| $C_4H_8$ | 81.6 | 0.5 |
| $C_5H_{12}$ | 0.4 | 74.4 |
| $C_5H_{10}$ | 0.1 | 24.6 |

Industrial Applicability

Aromatic hydrocarbon compounds can be produced stably over a long period of time and with high yield by using the zeolite-containing molded catalyst according to the present invention, which is a zeolite-containing molded catalyst for use in a process of producing aromatic hydrocarbon compounds by catalytic cyclization from a light hydrocarbon feedstock, to produce aromatic hydrocarbon compounds by bringing a light hydrocarbon feedstock into contact with the zeolite-containing molded catalyst in a reactor. Such stable production with high yield over a long period of time can be accomplished by a simple method because the zeolite-containing molded catalyst used in the process according to the present invention is highly resistant to both coking deterioration and regeneration degradation. These features are extremely useful for industrial application to the production of aromatic hydrocarbon compounds.

Brief Description of the Drawings

FIG. 1 is a diagram of a catalytic cyclization reaction test apparatus used in an example of the present invention.
FIG. 2 is a graph showing changes in the reaction over time in Example 1 of the present invention and Comparative Examples 1 and 2.
FIG. 3 is a graph showing changes in the reaction over time in Example 3 of the present invention.
FIG. 4 is a graph showing changes in the reaction over time in Example 4 of the present invention.
FIG. 5 is a graph showing changes in the reaction over time in Example 3 of the present invention and Comparative Example 3.

**Claims**

1.  A zeolite-containing molded catalyst for use in a process of producing an aromatic hydrocarbon compound by catalytic cyclization from a light hydrocarbon feedstock, wherein the zeolite contained in the zeolite-containing molded catalyst fulfills the following conditions (1), (2) and (3):

(1) the zeolite is a medium pore diameter zeolite with a pore diameter of from 5 to 6.5 A;
(2) the zeolite has a primary particle diameter in a range of from 0.02 to 0.25 $\mu$m; and
(3) the zeolite contains at least one metal element selected from the group consisting of the metal elements belonging to Group IB in the periodic table, and wherein the zeolite-containing molded catalyst also comprises at least one element selected from the group consisting of the elements belonging to Groups IB, IIB, IIIB and VIII in the periodic table.

2. The catalyst according to Claim 1, wherein the zeolite contains silver.

3. The catalyst according to Claim 1 or 2, wherein the zeolite is an MFI zeolite.

4. The catalyst according to any one of Claims 1 to 3, wherein the zeolite-containing molded catalyst is heat treated at a temperature of 500°C or more in a presence of steam prior to contact with the light hydrocarbon feedstock.

5. A process for producing an aromatic hydrocarbon compound, comprising bringing a light hydrocarbon feedstock into contact with a zeolite-containing molded catalyst according to any one of Claims 1 to 4.

6. The process according to Claim 5, wherein a reactor used in the process is an adiabatic fixed-bed reactor.

# FIG.1

CATALYTIC CYCLIZATION REACTION APPARATUS

EVAPORATOR 5

ELECTRIC FURNACE 6

NITROGEN OR AIR

WATER

REACTOR TUBE 7

GAS CHROMATOGRAPHY SAMPLING LINE 10

COOLER 9

FEEDSTOCK TANK 1

FEEDSTOCK TANK 2

ELECTRIC FURNACE 8

PRESSURE CONTROL VALVE 12

GAS-LIQUID SEPARATOR 11

PUMP 3

PUMP 4

EP 1 980 319 A1

# FIG.2

GRAPH OF CHANGES OVER TIME IN HEXANE/HEXENE CATALYTIC
CYCLIZATION REACTION USING CATALYSTS A-1, B-1 AND C-1

Legend:
- --▲-- CATALYST A-1
- --○-- CATALYST B-1
- --■-- CATALYST C-1

Y-axis: HEXA CONVERSION RATE (wt%)
X-axis: REACTION TIME (Hr)

EP 1 980 319 A1

# FIG.3

C$_4$ AND C$_5$ FRACTION CATALYTIC CYCLIZATION REACTION USING CATALYST A-1: GRAPH OF CHANGES OVER TIME IN REACTION/REGENERATION CYCLE

C$_{6-9}$ AROMATIC YIELD IS INDICATED AS WT% OF C$_{6-9}$ AROMATIC HYDROCARBON COMPOUNDS IN REACTION PRODUCT.
A 72-HOUR REACTION/20-HOUR REGENERATION CYCLE WAS REPEATED, BUT ONLY REACTION TIME IS PLOTTED.

EP 1 980 319 A1

# FIG.4

C$_4$ AND C$_5$ FRACTION CATALYTIC CYCLIZATION REACTION USING CATALYST F-1: GRAPH OF CHANGES OVER TIME

Legend: CATALYST F-1

Y-axis: C$_{6-9}$ AROMATIC YIELD (wt%), marked 0, 20, 40, 60

X-axis: REACTION TIME (days), marked 0, 6, 12, 18

C$_{6-9}$ AROMATIC YIELD IS INDICATED AS WT% OF C$_{6-9}$ AROMATIC HYDROCARBON COMPOUNDS IN REACTION PRODUCT.
A 72-HOUR REACTION/20-HOUR REGENERATION CYCLE WAS REPEATED, BUT ONLY REACTION TIME IS PLOTTED.

EP 1 980 319 A1

# FIG.5

C$_4$ AND C$_5$ FRACTION CATALYTIC CYCLIZATION REACTION USING CATALYST A-1 AND B-1: GRAPH OF CHANGES OVER TIME IN ONE CYCLE (THREE DAYS)

C$_{6-9}$ AROMATIC YIELD IS INDICATED AS WT% OF C$_{6-9}$ AROMATIC HYDROCARBON COMPOUNDS IN REACTION PRODUCT.

EP 1 980 319 A1

<div align="center">**INTERNATIONAL SEARCH REPORT**</div>

| | International application No. |
|---|---|
| | PCT/JP2007/050907 |

**A. CLASSIFICATION OF SUBJECT MATTER**
*B01J29/44*(2006.01)i, *B01J35/10*(2006.01)i, *B01J37/10*(2006.01)i, *C07C5/393*
(2006.01)i, *C07C15/04*(2006.01)i, *C10G35/095*(2006.01)i, *C07B61/00*
(2006.01)n

According to International Patent Classification (IPC) or to both national classification and IPC

**B. FIELDS SEARCHED**

Minimum documentation searched (classification system followed by classification symbols)
B01J29/44, B01J35/10, B01J37/10, C07C5/393, C07C15/04, C10G35/095,
C07B61/00

Documentation searched other than minimum documentation to the extent that such documents are included in the fields searched
Jitsuyo Shinan Koho          1922-1996    Jitsuyo Shinan Toroku Koho    1996-2007
Kokai Jitsuyo Shinan Koho    1971-2007    Toroku Jitsuyo Shinan Koho    1994-2007

Electronic data base consulted during the international search (name of data base and, where practicable, search terms used)
JST7580(JDream2)

**C. DOCUMENTS CONSIDERED TO BE RELEVANT**

| Category* | Citation of document, with indication, where appropriate, of the relevant passages | Relevant to claim No. |
|---|---|---|
| Y | WO 1996/013331 A1  (Asahi Chemical Industry Co., Ltd.),<br>09 May, 1996 (09.05.96),<br>Claims; page 6, lines 9 to 26; page 7, lines 16 to 19; page 9, lines 2 to 13; page 9, lines 19 to 22; examples 1 to 19<br>& US 5968342 A           & EP 788838 A1<br>& DE 69534721 T         & CN 1162274 A<br>& ZA 9508907 A          & RU 2133639 C<br>& KR 233194 B | 1-6 |

☒ Further documents are listed in the continuation of Box C.    ☐ See patent family annex.

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

Date of the actual completion of the international search
22 March, 2007 (22.03.07)

Date of mailing of the international search report
03 April, 2007 (03.04.07)

Name and mailing address of the ISA/
Japanese Patent Office

Authorized officer

Facsimile No.

Telephone No.

Form PCT/ISA/210 (second sheet) (April 2005)

**INTERNATIONAL SEARCH REPORT**

International application No.

PCT/JP2007/050907

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 3-16644 A  (The British Petroleum Co. p.l.c), 24 January, 1991 (24.01.91), Claims; page 3, upper right column, lines 7 to 10; page 3, lower left column, lines 9 to 16 & US 4990715 A          & US 5093294 A & EP 327764 A1          & GB 8803112 A & DE 3869119 C          & NZ 227423 A & CA 1329388 A | 1-6 |
| Y | JP 62-285987 A  (Keishitsu Ryubun Shin Yoto Kaihatsu Gijutsu Kenkyu Kumiai), 11 December, 1987 (11.12.87), Page 2, upper right column, lines 6 to 19; page 2, lower right column, line 15 to page 3, upper left column, line 11; example, catalyst (1,5) (Family: none) | 1-6 |
| Y | JP 6-346063 A  (Asahi Chemical Industry Co., Ltd.), 20 December, 1994 (20.12.94), Claim 1; Par. Nos. [0022] to [0024]; examples 1 to 5 (Family: none) | 1-6 |
| Y | JP 5-310415 A  (Asahi Chemical Industry Co., Ltd.), 22 November, 1993 (22.11.93), Claim 1; Par. Nos. [0014], [0043]; example 5 & US 5268162 A          & EP 514715 A1 & CA 2068670 A          & CN 1067226 A | 1-6 |

Form PCT/ISA/210 (continuation of second sheet) (April 2005)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S59117584 B
- JP H10052646 B
- WO 199613331 A

**Non-patent literature cited in the description**

- CRC Handbook of Chemistry and Physics. CRC Press Inc, 1994, 1-15
- The Society of Chemical Engineers. Chemical Engineering Handbook. 233
- *JCPDS 5-0669NBS Circ.,* 1953, vol. II, 38